# EUROPEAN PATENT APPLICATION

(11) **EP 2 465 488 A1**
(43) Date of publication of application: **20.06.2012**
(21) Application number: 10808071.4
(22) Date of filing: 03.08.2010
(51) Int. Cl.: A61K 8/97, A61K 36/18, A61P 17/00, A61P 17/16, A61P 39/06, A61Q 19/00, A61Q 19/02, A61Q 19/08

(54) **PREPARATION FOR EXTERNAL APPLICATION TO SKIN, SKIN WHITENING AGENT, ANTIOXIDANT AGENT, AND ANTI-AGING AGENT**

(30) Priority: 11.08.2009 JP 2009186478
(71) Applicant: Shiseido Company, Ltd., Chuo-ku Tokyo 104-8010 (JP)
(72) Inventor: SUZUKI, Rikako, Yokohama-shi Kanagawa 224-8558 (JP); HASEGAWA, Kiyotaka, Yokohama-shi Kanagawa 224-8558 (JP); SATO, Kiyoshi, Yokohama-shi Kanagawa 224-8558 (JP); KUSAKARI, Ken, Yokohama-shi Kanagawa 224-8558 (JP); YOKOI, Tokiya, Yokohama-shi Kanagawa 224-8558 (JP)
(74) Representative: Ulmann, Catherine Claire
(86) International application number: PCT/JP2010/004877
(87) International publication number: WO 2011/018885

(57) **Abstract**

Provided are a whitener having superior whitening action and superior skin lightening and whitening effects against pigment deposition, age spots, freckles, liver spots and the like, and an antioxidant and anti-aging agent having superior free radical capturing abilities that prevent and/or inhibit skin aging and skin diseases caused by active oxygen (free radicals). The whitener, antioxidant and anti-aging agent contain an extract from Polyalthia longifolia.

## Description

### TECHNICAL FIELD

The present invention relates to a whitener, an antioxidant and an anti-aging agent that comprise a plant extract as an active ingredient thereof, and more particularly, to an external skin preparation containing the same.

### BACKGROUND ART

Although there are some aspects of the mechanism by which wrinkles and the like form in skin that are unclear, it is generally thought that hormone abnormalities and excessive exposure to ultraviolet rays from sunlight cause the formation of melanin pigment that is abnormally deposited in the skin. This melanin pigment, which is responsible for skin coloring, is produced in melanin-forming granules (melanosomes) within melanin-producing cells (melanocytes) present between the epidermis and derma, and melanin that has been formed is transferred to keratinocytes and accumulates in the epidermis.

The biochemical reactions that occur within these melanocytes is presumed to consist of the reactions described below. Namely, a process by which the essential amino acid, tyrosine, is converted from dopa to dopaquinone by the action of the enzyme tyrosinase, and is subsequently transformed to black melanin by an enzymatic or non-enzymatic oxidative action constitutes the process by which melanin pigment is formed.
Whiteners inhibit this formation of melanin pigment, and various plant-derived extracts have previously been developed with the expectation of being safe as well as demonstrating only mild irritation of the skin (see, for example, Patent Documents 1 to 5).

On the other hand, active oxygen is known to be produced by ultraviolet rays. When the free radical form of active oxygen reacts with an oxidizing substance such as lipids, it induces an oxidizing chain reaction. Thus, active oxygen in the form of free radicals increases damage to skin and other body tissue.

Since the skin is constantly exposed to oxygen and ultraviolet rays, it is subjected to the greatest amount of damage in the form of oxidative stress caused by free radicals. In recent years, various forms of active oxygen generated by ultraviolet rays have been determined to cause peroxidation of skin and lipids, protein denaturation and enzyme disorders, and in the short term, induces skin inflammation and other disorders. In addition, this is thought to cause skin aging and cancer in the long term.

In addition, active oxygen and lipid peroxides are thought to be involved in skin diseases such as atopic dermatitis, contact dermatitis and psoriasis. In this manner, active oxygen (free radicals) is intimately involved in skin aging and skin diseases.

Substances that have the ability to capture free radicals are able to inhibit or interrupt radical chain reactions, and are equivalent to substances referred to as antioxidants.
Thus, external skin preparations that incorporate antioxidants can be expected to demonstrate preventive and/or ameliorative effects against skin aging (in the form of, for example, age spots, wrinkles or sagging) caused by photooxidative stress. In addition, external skin preparations for treatment of various types of skin diseases associated with free radicals can also be expected to demonstrate such preventive and/or ameliorative effects.

Vitamin E and vitamin C, which are known to be antioxidants, are antioxidative substances that capture free radicals in the body. In addition, synthetic antioxidative substances are also known in the manner of BHT and BHA. In addition, reported examples of plant-derived antioxidants include extracts of Lentinula edodes, Flammulina velutipes, Lyophyllum shimeji, Coriolus versicolor, Tricholoma matsutake, Ganoderma lucidum, Daedalea dickinsii, Pholiota nameko and other basidiomycetes (Patent Documents 6 to 8). Moreover, antioxidants composed of extracts of plants of the family Scrophulariaceae, genus Verbascum (Patent Document 9), and antioxidants composed of extracts of plants of the family Boraginaceae, family Cordia (Patent Document 10) have also been reported.

### Prior Art Documents

### Patent Documents

Patent Document 1: Japanese Unexamined Patent Publication No. H8-310939
Patent Document 2: Japanese Unexamined Patent Publication No. H7-89843
Patent Document 3: Japanese Unexamined Patent Publication No. H9-30954
Patent Document 4: Japanese Unexamined Patent Publication No. 2003-73224
Patent Document 5: Japanese Unexamined Patent Publication No. H9-263534
Patent Document 6: Japanese Unexamined Patent Publication No. H5-317016
Patent Document 7: Japanese Unexamined Patent Publication No. H6-65575
Patent Document 8: Japanese Unexamined Patent Publication No. S59-124984
Patent Document 9: Japanese Unexamined Patent Publication No. H11-171723
Patent Document 10: Japanese Unexamined Patent Publication No. H11-171720

### SUMMARY OF THE INVENTION

### Problems to be Solved by the Invention

In this manner, with respect to plant-derived whiteners, antioxidants and anti-aging agents, there is a need to find a novel plant that demonstrates novel whitening effects. Therefore, an object of the present invention is to accommodate these circumstances of the prior art and provide a novel plant-derived whitener, antioxidant and anti-aging agent as well as an external skin preparation that incorporates the same.

### Means for Solving the Problems

As a result of conducting extensive studies with the foregoing in view, the inventors of the present invention found that a specific plant extract has superior whitening action, antioxidative action and anti-aging action, thereby leading to completion of the present invention.

The present invention is an external skin preparation comprising an extract from Polyalthia longifolia.

The present invention is a whitening agent comprising an extract from Polyalthia longifolia.

The present invention is a whitening cosmetic comprising an extract from Polyalthia longifolia.

The present invention is an antioxidant comprising an extract from Polyalthia longifolia.

The present invention is an anti-aging agent comprising an extract from Polyalthia longifolia.

The present invention is an anti-aging cosmetic comprising an extract from Polyalthia longifolia.

### Effects of the Invention

The external skin preparation of the present invention has superior whitening action, anti-aging action and antioxidative action, and together with having superior skin lightening and whitening effects against pigment deposition, age spots, freckles, liver spots and the like, has effects that prevent and/or inhibit skin aging and skin diseases caused by active oxygen (free radicals).
The whitener of the present invention has superior whitening action, and has superior skin lightening and whitening effects against pigment deposition, age spots, freckles, liver spots and the like.
The whitening cosmetic of the present invention demonstrates superior whitening effects by being applied to the skin, has superior skin lightening and whitening effects against pigment deposition, age spots, freckles, liver spots and the like, and has a high degree of safety.
The anti-aging agent and antioxidant of the present invention have superior free radical capturing abilities that prevent and/or inhibit skin aging and skin diseases caused by active oxygen (free radicals).
The anti-aging cosmetic of the present invention demonstrates superior free radical capturing ability by being applied to the skin, is able to prevent and/or inhibit skin aging and skin diseases caused by active oxygen (free radicals), and has a high degree of safety.

### BRIEF DESCRIPTION OF THE DRAWINGS

FIG. 1 is a graph showing the results of evaluating the radical quenching action of ethanol extracts of Polyalthia longifolia.
FIG. 2 is a graph showing the results of evaluating the radical quenching action of 1,3-butanediol extracts of Polyalthia longifolia.

### EMBODIMENTS OF THE INVENTION

The following provides a detailed description of the present invention. The plant used in the present invention is Polyalthia longifolia belonging to the genus Polyalthia of the family Annonaceae. This Polyalthia longifolia belonging to the genus Polyalthia of the family Annonaceae is a tall evergreen tree that reaches a height of 15 to 20 meters and is planted in tropical regions as roadside trees and shade trees.

The whitening action, antioxidative action and anti-aging action of the plant of the present invention and extracts have heretofore not been known, and were first discovered by the inventors of the present invention.
In addition, there have also been no reports describing the use of the plant used in the present invention for external use.

The plant extract used in the present invention is obtained by suitably drying or crushing a leaf, branch, flower, bark, seed, fruit or root of the aforementioned plant followed by extracting with a solvent. Although extraction may be carried out while allowing to stand undisturbed at room temperature, extraction can be promoted by heating, stirring or refluxing while heating as necessary. The resulting liquid extract can be used as is or can be used after suitably subjecting to treatment such as filtration, concentration or decolorization. In addition, after having first removed the solvent, the extract can also be used by redissolving in a solvent differing from the solvent used for extraction. The resulting extract can also be used after further purifying by active charcoal, column chromatography and the like.

Although there are no particular limitations on the site used provided it does not impair the effects of the present invention, an extract from a leaf or branch is preferable.

The extraction solvent used in the present invention can be any solvent provided it is a solvent that is routinely used for extraction, and in particular, although organic solvents such as alcohols in the manner of methanol, ethanol, 1,3-butanediol, propylene glycol or dipropylene glycol, hydrated alcohols, acetone, ethyl acetate or chloroform can be used alone or in combination, methanol, ethanol, 1,3-butanediol and acetone are preferable, while methanol, ethanol and 1,3-butanediol are particularly preferable. In addition, these extracts may also be used after further purifying by solvent fractionation, active charcoal treatment or column chromatography and the like.

Although the whitener, antioxidant and anti-aging agent of the present invention are **characterized in that** they contain an extract from Polyalthia longifolia belonging to the genus Polyalthia of the family Annonaceae, various other components can also be contained within a range that does not impair the effects of the present invention.

The whitener, antioxidant and anti-aging agent of the present invention can be incorporated in an external skin preparation base to obtain a whitening external skin preparation or an anti-aging external skin preparation based on the antioxidative action thereof. These external skin preparations can be used preferably particularly in the fields of cosmetics, pharmaceuticals and quasi drugs.

The incorporated amount of plant extract in the external skin preparation of the present invention is normally 0.00001% by weight or more and preferably 0.0001% by weight or more (and this indicates the amount incorporated as a dried product obtained by removing the solvent unless specifically indicated otherwise). If the incorporated amount is excessively low, effects are not adequately demonstrated. Although there are no particular limitations on the upper limit of the incorporated amount provided it is within a range that does not impair the effects of the present invention, since remarkable effects corresponding to the increased amount are not obtained even if incorporated in excess, and there may also be detrimental effects in terms of drug design, ease of use and the like, the upper limit of the incorporated amount is normally 10% by weight or less and more preferably 5% by weight or less.

The external skin preparation of the present invention is produced by incorporating the whitener, antioxidant and anti-aging agent according to the present invention in a base of an external skin preparation. In addition to the aforementioned essential components, the external skin preparation of the present invention can also suitably incorporate, for example, a moisturizer, oxidation preventive, oily component, ultraviolet absorber, surfactant, thickener, alcohol, powder component, colorant, aqueous component, water, plant extract or various types of skin nutrients as necessary.

In addition, sequestering agents such as sodium edetate, trisodium edetate, sodium citrate, sodium polyphosphate, sodium metaphosphate or gluconic acid, drugs such as caffeine, tannin, verapamil, tranexamic acid and derivatives thereof, licorice extract, glabridin, fruit and hot water extract of Pyracantha crenulata, various types of herbal medicines, tocopherol acetate or glycyrrhizinic acid, derivatives thereof or salts thereof, other whiteners such as vitamin C, magnesium ascorbyl phosphate, ascorbic acid glucoside, albutin, kojic acid, alkoxysalicylic acid and/or salts thereof, and sugars such as glucose, fructose, mannose, sucrose or trehalose.

The drug form of the external skin preparation of the present invention may be any drug form conventionally used for external skin preparations, such as an ointment, cream, milky lotion, lotion, skin pack or bath additive, and there are no particular limitations thereon. Examples

Although the following provides a more detailed description of the present invention by listing examples thereof, the present invention is not limited thereto. Incorporated amounts are indicated in percent by weight (wt%) unless specifically indicated otherwise.
An explanation is first provided of a method of preparing the plant extract used in the present example, a method for testing melanin formation inhibitory effects and results thereof, and a method for testing antioxidative effects and results thereof.

### 1. Sample Preparation

Although all of the plants used in the present example were obtained in Okinawa, they are not limited thereto.
(1) 83 mL of methanol were added to 8.21 g of leaves and branches of Polyalthia longifolia followed by immersing for 7 days at room temperature and filtering. The methanol present in the filtrate was distilled off followed by drying under reduced pressure to obtain 1.385 g of a methanol extract.
(2) 10 mL of ethanol were added to 1.01 g of leaves and branches of Polyalthia longifolia followed by immersing for 7 days at room temperature and filtering. The ethanol present in the filtrate was distilled off followed by drying under reduced pressure to obtain 0.074 g of an ethanol extract.
(3) 10 mL of 1,3-butanediol were added to 1.00 g of leaves and branches of Polyalthia longifolia followed by immersing for 7 days at room temperature and filtering to obtain 7.05 g of a 1,3-butanediol extract. The amount of dry residue in the liquid extract was calculated to be 2.5 mg/g by measuring the weight after drying for 5 hours in a vacuum at 105°C.
(4) 0.6 g of the methanol extract obtained in (1) were dispersed and/or dissolved in 30 ml of water followed by the addition of 30 ml of ethyl acetate and extracting by vigorously mixing for several minutes in a separatory funnel. After allowing to stand undisturbed for several minutes, the ethyl acetate phase was recovered. The aqueous phase was further similarly extracted twice with ethyl acetate. The resulting ethyl acetate layers were then mixed followed by removing the ethyl acetate under reduced pressure to obtain about 0.31 g of an ethyl acetate fraction.

### 2. Method for Testing Inhibitory Effects on Melanin Formation and Results Thereof

The inhibitory effects on melanin formation were measured and evaluated according to the following method using the Polyalthia longifolia extracts obtained according to each of the previously described extraction methods as test samples.

### (1) Cell Seeding and Addition of Test Substance

Mouse B16 melanoma cells were disseminated into a 6-well plate at 100,000 cells/well. Test substance solutions (solvent: DMSO) were added on the following day.

### (2) Measurement of Cell Proliferation

The number of cells was measured based on cellular respiratory activity. After removing the medium by aspiration 3 days after addition of the test substance solutions, respiratory activity was measured under conditions of a reaction time of 30 minutes and temperature of 37°C using Alamar Blue reagent (Alamar Corp.). The ratio (%) of the number of cells in the sample substance addition groups to the number of cells of a control group to which only solvent was added was calculated. A higher cell ratio indicates lower cytotoxicity.

### (3) Measurement of Melanin Levels and Results

After aspirating off the medium and washing three times using a buffer (phosphate buffer, 50 mM, pH 6.8), 1 M NaOH was added to lyse the cells followed by measurement of optical absorbance at 475 nm. Since this optical absorbance is known to be proportional to the amount of melanin, measurement values were treated as relative values of the amount of melanin, and the ratios (%) of the amount of melanin of test substance addition groups to a control group (addition of solvent only) were calculated. A lower value for the ratio of the amount of melanin indicates higher inhibitory effects on melanin formation.

The results of the ratios of the amount of melanin (%) and the ratios of the number of cells (%) as determined using a methanol extract and ethanol extract of Polyalthia longifolia are shown in Table 1, while the results of the ratios of the amount of melanin (%) and the ratios of the number of cells (%) as determined using an ethyl acetate fraction of a Polyalthia longifolia methanol extract are shown in Table 2.

**[Table 1]**

| Polyalthia longifolia extract | 0 ppm | | 0.3 ppm | | 1 ppm | | 3 ppm | | 10 ppm | |
|---|---|---|---|---|---|---|---|---|---|---|
| | Amt. of melanin | Cell count | Amt. of melanin | Cell count | Amt. of melanin | Cell count | Amt. of melanin | Cell count | Amt. of melanin | Cell count |
| Methanol extract | 100 | 100 | 92 | 96 | 87 | 100 | 54 | 100 | 31 | 98 |
| 100% ethanol extract | 100 | 100 | 94 | 94 | 93 | 96 | 76 | 97 | 30 | 92 |

**[Table 2]**

| Polyalthia longifolia extract | 0 ppm | | 0.3 ppm | | 1 ppm | | 3 ppm | |
|---|---|---|---|---|---|---|---|---|
| | Amt. of melanin | Cell count | Amt. of melanin | Cell count | Amt. of melanin | Cell count | Amt. of melanin | Cell count |
| Ethyl acetate fraction of methanol extract | 100 | 100 | 96 | 102 | 86 | 101 | 54 | 106 |

Table 3 indicates the results of the ratio of the amount of melanin (%) and the ratio of the number of cells (%) as determined using a 1,3-butanediol extract of Polyalthia longifolia.
The 1,3-butanediol extract of Polyalthia longifolia was added to the medium to a final concentration of 0.4% (v/v). The solvent (1,3-butanediol) contained in the extract was added at 0.4% for use as a control. Cytotoxicity was not observed in either case.

**[Table 3]**

| | Amount of Melanin |
|---|---|
| Control | 100 |
| 100% butanediol | 43 |

According to Tables 1 to 3, the Polyalthia longifolia of the present invention demonstrated superior melanin inhibitory action for each of the methanol extract, ethanol extract, ethyl acetate fraction of a methanol extract and 1,3-butanediol extract, and was determined to be useful as a whitener.

### 3. Method for Testing Antioxidative Effects and Results Thereof

The plant extract used in the present invention also has superior antioxidative effects in addition to the aforementioned whitening action. The following provides a description of a method for testing antioxidative effects and the results thereof.

### (1) Evaluation of Antioxidative Effects (DPPH Radical Quenching Assay)

A test substance dissolved in dimethyl sulfoxide was injected into a 96 well plate in aliquots of 10 µl/well, followed by the addition of a 1 mmol/l 1,1-diphenyl-2-picrylhydrazyl solution at 90 µl/well. After allowing to stand for 10 minutes at room temperature, optical absorbance at 517 nm was measured and the radical quenching rate (%) versus a control was determined from the optical absorbance data.

### (2) Results of Evaluation of Antioxidative Effects

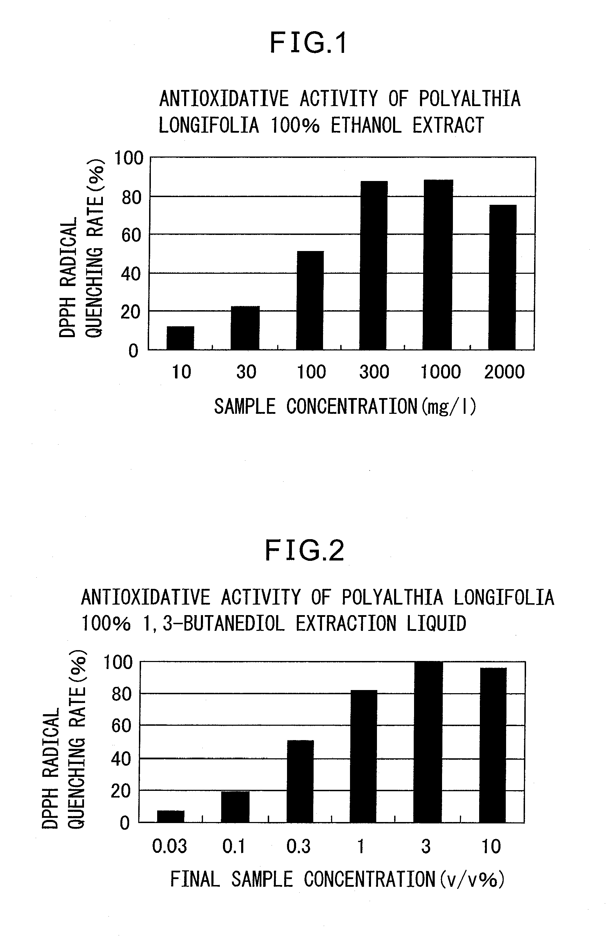

The evaluation results when using a 100% ethanol extract for the test substance are shown in FIG. 1. An ethanol extract prepared from Polyalthia longifolia was observed to demonstrate concentration-dependent DPPH radical quenching action starting at a low concentration, and was determined to have high antioxidative effects.

In addition, an extract obtained by extracting with 1,3-butanediol, which is a moisturizer (polyol) commonly used in cosmetics, that was used as a test substance also was observed to demonstrate high antioxidative effects as shown in FIG. 2.
On the basis of the above results, Polyalthia longifolia extract can be used as an anti-aging agent that demonstrates superior antioxidative effects, prevents skin aging and maintains the skin in a youthful, healthy state.

The following provides an explanation of examples of formations of various forms of external skin preparations according to the present invention. The content of Polyalthia longifolia extract in the formulas is indicated as the dry residue remaining after removing the extraction solvent.

| Formulation Example 1 - Beauty Lotion | |
|---|---|
| | wt% |
| Trimethylglycine | 1.0 |
| Polyalthia longifolia ethanol extract | 0.00001 |
| Glycerin | 1.0 |
| 1,3-butanediol | 5.0 |
| Sodium alginate | 0.1 |
| Ethyl alcohol | 5.0 |
| Polyoxyethylene polyoxypropylene decyl tetradecyl ether | 0.2 |
| Sodium hexametaphosphate | As suitable |
| Citric acid | As suitable |
| Sodium citrate | As suitable |
| Phenoxyethanol | As suitable |
| Fragrance | As suitable |
| Purified water | Balance |

| Formulation Example 2 - Beauty Lotion | |
|---|---|
| | wt% |
| Polyalthia longifolia acetone extract redissolved in 1,3-butanediol | 0.5 |
| Glycerin | 2.0 |
| 1,3-butanediol | 4.0 |
| Polyoxyethylene methyl chloride | 1.0 |
| PEG/PPG-14/7 dimethyl ether | 3.0 |
| Erythritol | 1.0 |
| Polyoxyethylene-hydrogenated castor oil | 0.5 |
| Polyglyceryl diisostearate | 0.3 |
| Triethylhexanoin | 0.3 |
| Trisodium EDTA | As suitable |
| Citric acid | As suitable |
| Sodium citrate | As suitable |
| Phenoxyethanol | As suitable |
| Purified water | Balance |

| Formulation Example 3 - Beauty Lotion | |
|---|---|
| | wt% |
| Tranexamic acid | 1.0 |
| Sodium 4-methoxysilicylate | 1.0 |
| Lipoic acid | 0.1 |
| Witch hazel leaf extract | 0.1 |
| Lipotaurine | 0.1 |
| Sophora angustifolia extract | 0.1 |
| Peach kernel extract | 0.1 |
| Beech bud extract | 0.1 |
| Polyalthia longifolia 1,3-butanediol extract | 0.0001 |
| Magnesium ascorbyl phosphate | 0.1 |
| Thiotaurine | 0.1 |
| Green tea extract | 0.1 |
| Peppermint extract | 0.1 |
| Iris root extract | 1.0 |
| Trimethylglycine | 1.0 |
| Glycerin | 1.0 |
| 1,3-butanediol | 5.0 |
| Hydroxyethyl cellulose | 0.05 |
| Ethyl alcohol | 5.0 |
| Polyoxyethylene polyoxypropylene decyl tetradecyl ether | 0.2 |
| Trisodium EDTA | As suitable |
| Citric acid | As suitable |
| Sodium citrate | As suitable |
| Phenoxyethanol | As suitable |
| Fragrance | As suitable |
| Purified water | Balance |

| Formulation Example 4 - Milky Lotion | |
|---|---|
| | wt% |
| Dipotassium glycyrrhizinate | 0.05 |
| Tocopherol acetate | 0.5 |
| Polyalthia longifolia propylene glycol extract | 0.001 |
| Sodium L-glutamate | 0.05 |
| Fennel extract | 0.1 |
| Yeast extract | 0.1 |
| Rehmannia root extract | 0.1 |
| Hydroxypropyl-β-cyclodextrin | 0.1 |
| Glycerin | 6.0 |
| 1,3-butanediol | 5.0 |
| Polyoxyethylene methyl chloride | 3.0 |
| Sunflower oil | 1.0 |
| Squalane | 2.0 |
| Isododecane | 4.0 |
| Dimethylpolysiloxane | 3.0 |
| Xanthan gum | 0.1 |
| Carboxyvinyl polymer | 0.1 |
| Acrylic acid-alkyl methacrylate copolymer | 0.1 |
| Ethyl alcohol | 5.0 |
| Potassium hydroxide | As suitable |
| Sodium hexametaphosphate | As suitable |
| Red iron oxide | As suitable |
| Yellow iron oxide | As suitable |
| Ethyl p-hydroxybenzoate | As suitable |
| Fragrance | As suitable |
| Purified water | Balance |

| Formulation Example 5 - Daytime Milky Lotion | |
|---|---|
| | wt% |
| Dipotassium glycyrrhizinate | 0.1 |
| Polyalthia longifolia acetone extract redissolved in ethanol | 0.00003 |
| Tocopherol acetate | 0.1 |
| 1,3-butanediol | 5.0 |
| Squalane | 0.5 |
| Isododecane | 10.0 |
| Isohexadecane | 25.0 |
| Dimethylpolysiloxane | 2.0 |
| Polyoxyethylene-methylpolysiloxane copolymer | 1.5 |
| Trimethylsiloxysilicic acid | 1.0 |
| 4-t-butyl-4'-methoxydibenzoyl methane | 1.0 |
| 2-ethylhexyl paramethoxycinnamate | 5.0 |
| Glyceryl diparamethoxycinnamate | 1.0 |
| mono-2-ethylhexanoate | |
| Silicone-coated fine particulate titanium oxide | 4.0 |
| Dimethyldistearyl ammonium hectorite | 0.5 |
| Spherical polyethylene powder | 3.0 |
| Talc | 5.0 |
| Trisodium EDTA | As suitable |
| Phenoxyethanol | As suitable |
| Fragrance | As suitable |
| Purified water | Balance |

| Formulation Example 6 - Milky Lotion | |
|---|---|
| | wt% |
| L-arginine | 0.1 |
| Royal jelly extract | 0.1 |
| Yeast extract | 0.1 |
| Polyalthia longifolia 90% ethanol extract | 1.0 |
| Stearyl glycyrrhizinate | 0.05 |
| Tocopherol acetate | 0.1 |
| Sodium acetyl hyaluronate | 0.1 |
| Glycerin | 5.0 |
| Dipropylene glycol | 7.0 |
| Polyethylene glycol 1500 | 2.0 |
| Liquid paraffin | 7.0 |
| Vaseline | 3.0 |
| Behenyl alcohol | 1.0 |
| Batyl alcohol | 2.0 |
| Jojoba oil | 1.0 |
| Stearic acid | 0.5 |
| Isostearic acid | 0.5 |
| Behenic acid | 0.5 |
| Pentaerythritol tetra(2-ethylhexanoate) | 3.0 |
| Cetyl 2-ethylhexanoate | 3.0 |
| Glycerin monostearate | 1.0 |
| Polyoxyethylene glyceryl monostearate | 1.0 |
| Carboxyvinyl polymer | 0.15 |
| Sodium hexametaphosphate | As suitable |
| Potassium hydroxide | As suitable |
| Methyl p-hydroxybenzoate | As suitable |
| Fragrance | As suitable |
| Purified water | Balance |

| Formulation Example 7 - Milky Lotion | |
|---|---|
| | wt% |
| Ascorbic acid glucoside | 1.5 |
| Tranexamic acid | 1.0 |
| Tocopherol acetate | 0.1 |
| Sodium hyaluronate | 0.05 |
| Polyalthia longifolia 1,3-butanediol extract | 0.0003 |
| Pantothenyl ethyl ether | 0.1 |
| Stearyl glycyrrhizinate | 0.1 |
| Glycerin | 7.0 |
| 1,3-butanediol | 5.0 |
| Polyethylene glycol 2000 | 0.5 |
| Vaseline | 2.0 |
| Jojoba oil | 3.0 |
| Squalane | 2.0 |
| Phytostearyl hydroxystearate | 0.5 |
| Behenyl alcohol | 0.5 |
| Batyl alcohol | 0.2 |
| Dimethylpolysiloxane | 2.0 |
| Pentaerythritol tetra(2-ethylhexanoate) | 0.1 |
| Polyoxyethylene-hydrogenated castor oil | 1.0 |
| Polyoxyethylene glyceryl isostearate | 3.0 |
| 4-t-butyl-4'-dimethoxybenzoyl methane | 0.1 |
| Glyceryl diparamethoxycinnamate | 0.1 |
| mono-2-ethylhexanoate | |
| Xanthan gum | 0.1 |
| Carboxyvinyl polymer | 0.2 |
| Ethanol | 5.0 |
| Potassium hydroxide | As suitable |
| Sodium metabisulfite | As suitable |
| Sodium hexametaphosphate | As suitable |
| Trisodium EDTA | As suitable |
| Yellow iron oxide | As suitable |
| Paraoxybenzoic acid ester | As suitable |
| Purified water | Balance |

| | |
|---|---|
| Formulation Example 8 - Cream | |
| | wt% |
| Polyalthia longifolia 90% 1,3-butanediol extract | 0.05 |
| Potassium 4-methoxysalicylate | 3.0 |
| Propylene glycol | 5.0 |
| Glycerin | 8.0 |
| Stearic acid | 2.0 |
| Stearyl alcohol | 7.0 |
| Hydrogenated lanolin | 2.0 |
| Squalane | 5.0 |
| 2-octyldodecyl alcohol | 6.0 |
| Polyoxyethylene cetyl alcohol ether | 3.0 |
| Glyceryl monostearate | 2.0 |
| Potassium hydroxide | As suitable |
| Ethyl p-hydroxybenzoate | As suitable |
| Fragrance | As suitable |
| Ion exchange water | Balance |

| Formulation Example 9 - Cream | |
|---|---|
| | wt% |
| Potassium 4-methoxysalicylate | 1.0 |
| 3-O-ethyl ascorbic acid | 1.0 |
| Polyalthia longifolia 50% ethanol extract | 0.3 |
| Coenzyme Q10 | 0.03 |
| Tranexamic acid | 2.0 |
| Tocopherol acetate | 0.1 |
| Sodium hyaluronate | 0.05 |
| Pantothenyl ethyl ether | 0.1 |
| Stearyl glycyrrhizinate | 0.1 |
| Glycerin | 7.0 |
| 1,3-butanediol | 5.0 |
| Polyethylene glycol 20000 | 0.5 |
| Vaseline | 2.0 |
| Behenyl alcohol | 0.5 |
| Batyl alcohol | 0.2 |
| Squalane | 2.0 |
| Phytostearyl hydroxystearate | 0.5 |
| Jojoba oil | 3.0 |
| Pentaerythritol tetra(2-ethylhexanoate) | 1.0 |
| Dimethylpolysiloxane | 2.0 |
| Polyoxyethylene glyceryl isostearate | 1.5 |
| Polyoxyethylene-hydrogenated castor oil | 1.0 |
| Carboxyvinyl polymer | 0.2 |
| Xanthan gum | 0.1 |
| Ethanol | 5.0 |
| Sodium hexametaphosphate | As suitable |
| Yellow iron oxide | As suitable |
| Trisodium EDTA | As suitable |
| Potassium hydroxide | As suitable |
| Paraoxybenzoic acid ester | As suitable |
| Purified water | Balance |

| Formulation Example 10 - Two-Layer Daytime Milky Lotion | |
|---|---|
| | wt% |
| Tranexamic acid | 2.0 |
| Potassium 4-methoxysalicylate | 1.0 |
| Polyalthia longifolia 50% 1,3-butanediol extract | 0.3 |
| Dipotassium glycyrrhizinate | 0.02 |
| Glutathione | 1.0 |
| Thiotaurine | 0.05 |
| Sophora angustifolia extract | 1.0 |
| Dipropylene glycol | 5.0 |
| Dimethylpolysiloxane | 5.0 |
| Isohexadecane | 25.0 |
| Polyoxyethylene-methylpolysiloxane copolymer | 2.0 |
| Dimethyldistearyl ammonium hectorite | 0.5 |
| Butylethylpropanediol | 0.5 |
| 2-ethylhexyl paramethoxycinnamate | 7.5 |
| Trimethylsiloxysilicate | 5.0 |
| Spherical alkyl polyacrylate powder | 5.0 |
| Dextrin palmitate-coated fine particulate zinc oxide | 15.0 |
| Trisodium EDTA | As suitable |
| Methyl p-hydroxybenzoate | As suitable |
| Phenoxyethanol | As suitable |
| Fragrance | As suitable |
| Purified water | Balance |

| Formulation Example 11 - Gel | |
|---|---|
| | wt% |
| Potassium 4-methoxysalicylate | 0.1 |
| Lamium album extract | 0.1 |
| Polyalthia longifolia ethyl acetate fraction | 0.00001 |
| Dipotassium glycyrrhizinate | 0.1 |
| Ascorbic acid glucoside | 2.0 |
| Tocopherol acetate | 0.1 |
| Scutellaria baicalensis extract | 0.1 |
| Saxifraga sarmentosa extract | 0.1 |
| Glycerin | 2.0 |
| 1,3-butanediol | 5.0 |
| Polyethylene glycol 1500 | 3.0 |
| Polyethylene glycol 20000 | 3.0 |
| Powdered agar | 1.5 |
| Xanthan gum | 0.3 |
| Acrylic acid-alkyl methacrylate copolymer | 0.05 |
| Cetyl octanoate | 3.0 |
| Dimethylpolysiloxane | 5.0 |
| Sodium hexametaphosphate | As suitable |
| Dibutylhydroxytoluene | As suitable |
| Yellow iron oxide | As suitable |
| Citric acid | As suitable |
| Sodium citrate | As suitable |
| Sodium hydroxide | As suitable |
| Phenoxyethanol | As suitable |
| Fragrance | As suitable |
| Purified water | Balance |

## Claims

1. An external skin preparation comprising an extract from Polyalthia longifolia.

2. A whitening agent comprising an extract from Polyalthia longifolia.

3. A whitening cosmetic comprising an extract from Polyalthia longifolia.

4. An antioxidant comprising an extract from Polyalthia longifolia.

5. An anti-aging agent comprising an extract from Polyalthia longifolia.

6. An anti-aging cosmetic comprising an extract from Polyalthia longifolia.
